# EUROPEAN PATENT SPECIFICATION

(11) **EP 2 931 035 B1**
(45) Date of publication and mention of the grant of the patent: **08.03.2017**
(21) Application number: 13814061.1
(22) Date of filing: 04.12.2013
(51) Int. Cl.: A01N 25/00, A01N 25/30, A01N 57/20, C07C 217/08, A01P 13/00

(54) **COMPOSITION COMPRISING A PESTICIDE AND AN ALKOXYLATE OF 2-ISOPROPYL-5-METHYLHEXANE-1-AMINE**
ZUSAMMENSETZUNG, UMFASSEND EIN PESTIZID UND EIN ALKOXYLAT VON 2-ISOPROPYL-5-METHYLHEXAN-1-AMIN
COMPOSITION COMPRENANT UN PESTICIDE ET UN ALCOXYLATE DE 2-ISOPROPYL-5-MÉTHYLHEXANE-1-AMINE

(30) Priority: 14.12.2012 EP 12197160
(43) Date of publication of application: 21.10.2015
(73) Proprietor: BASF SE, 67056 Ludwigshafen am Rhein (DE)
(72) Inventor: BAUER, Frederic, 67146 Deidesheim (DE); KLINGELHOEFER, Paul, 68165 Mannheim (DE); WIGBERS, Christof Wilhelm, 68167 Mannheim (DE); REIS-WALTHER, Eva-Maria, 64747 Breuberg (DE)
(74) Representative: BASF IP Association
(86) International application number: PCT/EP2013/075450
(87) International publication number: WO 2014/090642

(56) References cited:
- WO-A1-2012/116939
- WO-A2-2007/030312
- WO-A2-2011/086115
- WO-A2-2011/101303
- US-A- 5 668 085

## Description

The present invention relates to a composition comprising a pesticide and an alkoxylate. The invention further relates to the alkoxylate, to a method for the production thereof and to the use thereof as adjuvant in pesticide-comprising spray mixtures. The invention further relates to a method for controlling phytopathogenic fungi and/or undesirable plant growth and/or undesirable insect or mite infestation and/or for regulating the growth of plants, wherein the composition is allowed to act on the respective pests, the habitat thereof or the plants to be protected from the respective pest, on the soil and/or on undesirable plants and/or the crop plants and/or the habitat thereof. Furthermore, the invention relates to seed comprising the composition.
The present invention comprises combinations of preferred features with other preferred features.
Alkoxylates and their use in agrochemical formulations as adjuvants are generally known:
WO 03/090531 describes the use of defined alkoxylates of the amphiphilic type, i.e. alkoxylates based on branched alcohols such as 2-propylheptanol, C₁₃-oxoalcohols and C₁₀-oxoalcohols, as activity-improving adjuvants in the agrotechnical field.
WO 11/086115 describes that alkoxylates of 2-propylheptylamine can be used as adjuvants in agrochemical formulations.
US 5 668 085 A describes glyphosate formulations comprising alkoxylated amine surfactants. WO 2007/030312 A2 describes agricultural compositions containing at least one amine ethoxylate and glycerol which enhance performance of herbicides. WO 2011/101303 A2 describes a compound comprising a pesticide and an alkoxylate of isoheptadecylamine. WO 2012/116939 A1 describes a composition comprising a pesticide, a surfactant and an alkoxylate of 2-propylheptylamine.Alkoxylated alkylamines, in particular commercially available ethoxylated tallow fatty amines (POEA), have important toxic properties (such as irritation of the skin and the eyes) and ecotoxic properties (such as high ecotoxicity to aquatic organisms such as algae and daphnias). Thus, for example, POEA (CAS No. 61791-26-2), which is frequently present in Roundup® herbicides as a wetter, is considered to be relatively toxic to aquatic organisms (Tsui and Chu, Chemosphere 2003, 52, 1189-1197).

It was therefore an object of the present invention to find an adjuvant which is well suited to herbicides such as glyphosate. Furthermore, the adjuvant should make possible a storage-stable formulation of the pesticides.

The object was solved by a composition comprising a pesticide and an alkoxylate, wherein the alkoxylate is an amine alkoxylate (A) 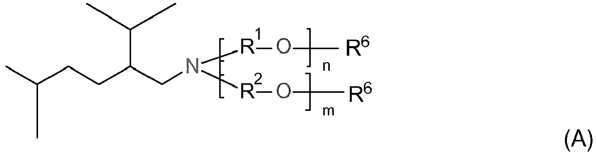 or a quaternized derivative (AQ) 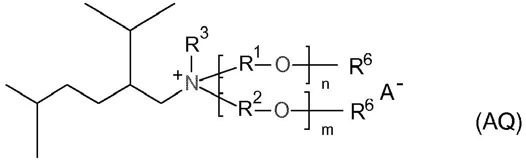 of the amine alkoxylate (A), where
R¹, R², and R⁵ independently of one another are ethylene, propylene, butylene or a mixture of these,
R³ is an H, -OH, -OR⁴, -[R⁵-O]ₚ-R⁶, C₁-C₆-alkyl or an oxygen anion,
R⁴ is a C₁-C₆-alkyl, C₂-C₆-alkenyl or C₂-C₆-alkynyl,
R⁶ is an H, C₁-C₆-alkyl, C₂-C₆-alkenyl, C₂-C₆-alkynyl, -SO₃R^{a}, -P(O)OR^{b}OR^{c}, -CH₂CO₂R^{d}, or -C(O)Re,
R^{a} and R^{d} independently of one another are an H, inorganic or organic cations,
R^{b} and R^{c} independently of one another are an H, inorganic or organic cations, C₁-C₆-alkyl, C₂-C₆-alkenyl or C₂-C₆-alkynyl,
R^{e} is C₁-C₂₂-alkyl, C₂-C₂₂-alkenyl, C₂-C₂₂-alkynyl, C₆-C₂₂-aryl or C₇-C₂₂-alkylaryl,
n, m and p independently of one another have a value of from 1 to 30,
A⁻ is an agriculturally acceptable anion, or, if R³ is an oxygen anion, A⁻ is absent.

Preferably, the composition according to the invention comprises a pesticide and an alkoxylate, wherein the alkoxylate is an amine alkoxylate (A).

Preferably, n has a value of from 1 to 20, especially preferably from 1 to 15. Preferably, m has a value of from 1 to 20, especially preferably from 1 to 15. Preferably, p has a value of from 1 to 30, especially preferably from 1 to 20. The values of n, m and p are normally average values as they mostly arise upon the alkoxylation with alkoxides. Therefore, n, m and p can not only be integers, but also all values between the integers.

Preferably, in the case of the amine alkoxylate (A), the total of n and m is 2 to 40 and in its quaternized derivative (AQ) the total of n, m and p is 3 to 80.

In the case of the amine alkoxylate (A) the total of n and m is especially preferably 3 to 30 and specifically 5 to 25. In a further especially preferred embodiment, the total of n and m is 6 to 9, in particular 6.5 to 8.5 and in particular 6.9 to 7.9. In a further especially preferred embodiment, the total of n and m is 11 to 40, in particular 12 to 30 and in particular 13.5 to 25. In a further especially preferred embodiment, the sum of n and m is 8 to 13, in particular 9 to 11.

In the case of the quaternized derivative (AQ) of the amine alkoxylate (A), the total of n, m and p is especially preferably 3 to 40 and specifically 5 to 25. In one especially preferred embodiment, the sum of n, m and p is 8 to 13, in particular 9 to 11.

R¹, R² and R⁵ are preferably independently of one another ethylene, ethylene and propylene, ethylene and butylene, or ethylene, propylene and butylene. In a further preferred embodiment, R¹, R² and R⁵ are propylene. In a further preferred embodiment, R¹, R² and R⁵ are butylene. Especially preferably R¹, R² and R⁵ independently of one another are ethylene, or ethylene and propylene. Very especially preferably, R¹, R² and R⁵ are ethylene.

If R¹, R² or R⁵ comprise a butylene radical, the latter may be present as a n-butylene, an isobutylene or a 2,3-butylene group, with n-butylene and isobutylene being preferred and n-butylene being most preferred.

R¹, R² and R⁵ independently of one another may be a mixture of ethylene, propylene or butylene. In this context, for example one or all radicals R¹, R² and R⁵ may comprise a mixture of these groups in each alkoxylate chain. Such mixtures can be linked to one another in any desired order, for example randomly or blockwise (such as one block ethylene and one block propylene). Also, it is possible for in each case one or more of the radicals R¹, R², and R⁵ to form a complete alkoxylate chain composed of different alkylene groups. For example, R¹ and R² may be composed of ethylene and R⁵ of propylene.

R³ is preferably an H, -OH, -OR⁴, -[R⁵-O]ₚ-R⁶, C₁-C₆-alkyl or an oxygen anion, it is especially preferably an H, methyl, butyl or an oxygen anion. In a specifically preferred embodiment, R³ is a methyl. In a further specifically preferred embodiment, R³ is an oxygen anion. In a further specifically preferred embodiment, R³ is an H.

R⁴ is preferably a C₁-C₆-alkyl, C₂-C₆-alkenyl or C₂-C₆-alkynyl, in particular a methyl or butyl, especially methyl.

R⁶ is preferably an H, C₁-C₆-alkyl, C₂-C₆-alkenyl, C₂-C₆-alkynyl, -SO₃R^{a}, -P(O)OR^{b}OR^{c}, -CH₂CO₂R^{d}, or -C(O)R^{e}, more preferably an H or methyl, especially more preferably H.

R^{a} and R^{d} are independently of one another H, or inorganic or organic cations, which may be singly or multiply positively charged. Examples of inorganic cations are cations of ammonium, Na⁺, K⁺, Mg²⁺, Ca²⁺, or Zn²⁺. Examples of organic cations are methylammonium, dimethylammonium, trimethylammonium, tetramethylammonium, (2-hydroxyethyl)ammonium, bis(2-hydroxyethyl)ammonium, tris(2-hydroxyethyl)-ammonium, tetra(2-hydroxyethyl)ammonium. Preferably, R^{a} and R^{d} are independently of one another H or inorganic cations. If an inorganic or organic cation is present, then the associated anionic group would be formed by the corresponding functional group (e.g., -SO₃⁻, -P(O)O⁻O⁻, or -CH₂CO₂⁻) on R⁶.

R^{b} and R^{c} are preferably, independently of one another, H, inorganic or organic cations C₁-C₆-alkyl, C₂-C₆-alkenyl or C₂-C₆-alkynyl. Suitable inorganic or organic cations are those specified for R^{a} above.

In another embodiment, in the quaternary derivative (AQ), the radicals R^{a}, R^{b}, R^{c} and R^{d} independently of one another may be organic cations, with the cationic group being the quaternary nitrogen cation of AQ itself. It would also be possible, therefore, for An to form a zwitterion, with the anionic group being formed by the corresponding functional group (e.g., -SO₃⁻, -P(O)O⁻O⁻, or -CH₂CO₂⁻) on R⁶ in AQ, and the cationic group by the quaternary nitrogen of AQ. In this zwitterionic form of AQ, the presence of an agriculturally acceptable anion A⁻ is optional.
R^{e} is preferably C₁-C₂₂-alkyl, C₂-C₂₂-alkenyl, C₂-C₂₂-alkynyl, C₆-C₂₂-aryl, or C₇-C₂₂-alkylaryl, more preferably C₁-C₆-alkyl.

A⁻ is an agriculturally acceptable anion, as they are generally known to the skilled person. Preferably, A⁻ is a halide (such as chloride or bromide), phosphate, sulfate or an anionic pesticide. Also carboxylates such as propionate, acetate, carbonate or formate are suitable as A⁻. Especially preferably, A⁻ is an anionic pesticide, such as a glyphosate anion or glufosinate anion. If R³ is an oxygen anion, an amine oxide is present. In this case, a further anion such as A⁻ is absent.

In the case of the amine alkoxylate (A), it is preferred that R¹ and R² independently of one another are ethylene, ethylene and propylene, ethylene and butylene, or ethylene, propylene and butylene, and the total of n and m is 2 to 60, preferably 2 to 40, especially preferably 3 to 30 and in particular 5 to 25. In a further preferred embodiment, R¹ and R² are ethylene, ethylene and propylene, ethylene and butylene, or ethylene, propylene and butylene and the total of n and m is 6 to 9, in particular 6.5 to 8.5 and in particular 6.9 to 7.9 In a further preferred embodiment, R¹ and R² are ethylene, ethylene and propylene, ethylene and butylene, or ethylene, propylene and butylene and the total of n and m is 11 to 40, in particular 12 to 30 and in particular 13.5 to 25. In one particularly preferred embodiment, R¹ and R² are ethylene, ethylene and propylene, ethylene and butylene, or ethylene, propylene and butylene, and the sum of n and m is 6 to 14, more particularly 8 to 12, and especially 9 to 11.

In the case of the amine alkoxylate (A), it is especially preferred that R¹ and R² are ethylene, and the total of n and m is 2 to 60, preferably 2 to 40, especially preferably 3 to 30, and in particular 5 to 25. In a further especially preferred embodiment, R¹ and R² are ethylene and the total of n and m is 6 to 9, in particular 6.5 to 8.5 and in particular 6.9 to 7.9. In a further especially preferred embodiment, R¹ and R² are ethylene and the total of n and m is 11 to 40, in particular 12 to 30 and in particular 13.5 to 25.

The compounds (A) and (AQ) may be present as mixtures of stereoisomers or as isolated stereoisomers. Tautomers and betaines are likewise encompassed by the structures (A) and (AQ).

In most cases, the composition according to the invention comprises from 0.1 to 90% by weight of the alkoxylate as defined above, preferably from 1 to 50% by weight and in particular from 3 to 30% by weight.

The term pesticide refers to at least one active substance selected from the group of the fungicides, insecticides, nematicides, herbicides, safeners, molluscicides, rodenticides and/or growth regulators. Preferred pesticides are fungicides, insecticides, herbicides and growth regulators. Especially preferred pesticides are herbicides and growth regulators. Mixtures of pesticides from two or more of the abovementioned classes may also be used. The skilled person is familiar with such pesticides, which can be found, for example, in Pesticide Manual, 14th Ed. (2006), The British Crop Protection Council, London. The above disclosed pesticides can be combined with any alkoxylate of the present invention. Suitable pesticides that can be combined with the alkoxlyates of the present invention are:
A) strobilurins:
   azoxystrobin, dimoxystrobin, coumoxystrobin, coumethoxystrobin, enestroburin, fluoxastrobin, kresoxim-methyl, metominostrobin, orysastrobin, picoxystrobin, pyraclostrobin, pyrametostrobin, pyraoxystrobin, pyribencarb, trifloxystrobin, methyl 2-[2-(2,5-dimethylphenyloxymethyl)phenyl]-3-methoxyacrylate, 2-(2-(3-(2,6-di-chlorophenyl)-1-methylallylideneaminooxymethyl)phenyl)-2-methoxyimino-N-methylacetamide;
B) carboxamides:
   - carboxanilides: benalaxyl, benalaxyl-M, benodanil, bixafen, boscalid, carboxin, fenfuram, fenhexamid, flutolanil, furametpyr, isopyrazam, isotianil, kiralaxyl, mepronil, metalaxyl, metalaxyl-M (mefenoxam), ofurace, oxadixyl, oxycarboxin, penflufen (N-(2-(1,3-dimethylbutyl)phenyl)-1,3-dimethyl-5-fluoro-1 H-pyrazole-4-carboxamide), penthiopyrad, sedaxane, tecloftalam, thifluzamide, tiadinil, 2-amino-4-methylthiazole-5-carboxanilide, N-(3',4',5'-trifluorobiphenyl-2-yl)-3-difluoromethyl-1-methyl-1 H-pyrazole-4-carboxamide, N-(4'-trifluoromethylthiobiphenyl-2-yl)-3-difluoromethyl-1-methyl-1 H-pyrazole-4-carboxamide, N-(2-(1,3,3-trimethylbutyl)-phenyl)-1,3-dimethyl-5-fluoro-1 H-pyrazole-4-carboxamide;
   - carboxylic acid morpholides: dimethomorph, flumorph, pyrimorph;
   - benzamides: flumetover, fluopicolide, fluopyram, zoxamid;
   - other carboxamides: carpropamid, diclocymet, mandipropamid, oxytetracyclin, silthiofam, N-(6-methoxypyridin-3-yl)cyclopropanecarboxamide;
C) azoles:
   - triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole;
   - imidazoles: cyazofamid, imazalil, imazalil sulfate, pefurazoate, prochloraz, triflumizole;
   - benzimidazoles: benomyl, carbendazim, fuberidazole, thiabendazole;
   - others: ethaboxam, etridiazole, hymexazole, 2-(4-chlorophenyl)-N-[4-(3,4-dimethoxyphenyl)isoxazol-5-yl]-2-prop-2-ynyloxyacetamide;
D) nitrogenous heterocyclyl compounds
   - pyridines: fluazinam, pyrifenox, 3-[5-(4-chlorophenyl)-2,3-dimethylisoxazolidin-3-yl]-pyridine, 3-[5-(4-methylphenyl)-2,3-dimethylisoxazolidin-3-yl]pyridine;
   - pyrimidines: bupirimate, cyprodinil, diflumetorim, fenarimol, ferimzone, mepanipyrim, nitrapyrin, nuarimol, pyrimethanil;
   - piperazines: triforine;
   - pyrroles: fludioxonil, fenpiclonil;
   - morpholines: aldimorph, dodemorph, dodemorph acetate, fenpropimorph, tridemorph;
   - piperidines: fenpropidin;
   - dicarboximides: fluorimid, iprodione, procymidone, vinclozolin;
   - nonaromatic 5-membered heterocyclic rings: famoxadon, fenamidon, flutianil, octhilinone, probenazole, S-allyl 5-amino-2-isopropyl-3-oxo-4-orthotolyl-2,3-dihydro-pyrazole-1-thiocarboxylate;
   - others: acibenzolar-S-methyl, amisulbrom, anilazin, blasticidin-S, captafol, captan, quinomethionate, dazomet, debacarb, diclomezine, difenzoquat, difenzoquat methylsulfate, fenoxanil, folpet, oxolinic acid, piperalin, proquinazid, pyroquilon, quinoxyfen, triazoxide, tricyclazole, 2-butoxy-6-iodo-3-propylchromen-4-one, 5-chloro-1-(4,6-dimethoxypyrimidin-2-yl)-2-methyl-1 H-benzimidazole, 5-chloro-7-(4-methylpiperidin-1-yl)-6-(2,4,6-trifluorophenyl)-[1,2,4]triazolo[1,5-a]pyrimidine, 5-ethyl-6-octyl-[1,2,4]triazolo[1,5-a]pyrimidin-7-ylamine;
E) carbamates and dithiocarbamates
   - thio- and dithiocarbamates: ferbam, mancozeb, maneb, metam, methasulphocarb, metiram, propineb, thiram, zineb, ziram;
   - carbamates: diethofencarb, benthiavalicarb, iprovalicarb, propamocarb, propamocarb hydrochloride, valiphenal, (4-fluorophenyl) N-(1-(1-(4-cyanophenyl)ethanesulfonyl)-but-2-yl)carbamate;
F) other fungicides
   - guanidines: dodine, dodine free base, guazatine, guazatine acetate, iminoctadine, iminoctadine triacetate, iminoctadine tris(albesilate);
   - antibiotics: kasugamycin, kasugamycin hydrochloride hydrate, polyoxins, streptomycin, validamycin A;
   - nitrophenyl derivatives: binapacryl, dicloran, dinobuton, dinocap, nitrothal-isopropyl, tecnazene;
   - organometallic compounds: fentin salts such as, for example, fentin acetate, fentin chloride, fentin hydroxide;
   - sulfurous heterocyclyl compounds: dithianon, isoprothiolane;
   - organophosphorus compounds: edifenphos, fosetyl, fosetyl-aluminum, iprobenfos, phosphorous acid and its salts, pyrazophos, tolclofos-methyl;
   - organochlorine compounds: chlorthalonil, dichlofluanid, dichlorphen, flusulfamide, hexachlorobenzene, pencycuron, pentachlorophenol and its salts, phthalide, quintozene, thiophanate-methyl, tolylfluanid, N-(4-chloro-2-nitrophenyl)-N-ethyl-4-methylbenzenesulfonamide;
   - inorganic active substances: phosphorous acid and its salts, Bordeaux mixture, copper salts such as, for example, copper acetate, copper hydroxide, copper oxychloride, basic copper sulfate, sulfur;
   - biological products for controlling fungi, plant strengthening products: *Bacillus subtilis* strain NRRL No. B-21661 (for example the products RHAPSODY^{®}, SERENADE^{®} MAX and SERENADE^{®} ASO from AgraQuest, Inc., USA.), *Bacillus pumilus* strain NRRL No. B-30087 (for example SONATA^{®} and BALLAD^{®} Plus from AgraQuest, Inc., USA), *Ulocladium oudemansii* (for example BOTRY-ZEN from BotriZen Ltd., New Zealand), chitosan (for example ARMOUR-ZEN from BotriZen Ltd., New Zealand).
   - others: biphenyl, bronopol, cyflufenamid, cymoxanil, diphenylamine, metrafenon, mildiomycin, oxine-copper, prohexadione-calcium, spiroxamin, tolylfluanid, N-(cyclopropylmethoxyimino-(6-difluoromethoxy-2,3-difluorophenyl)methyl)-2-phenyl-acetamide, N'-(4-(4-chloro-3-trifluoromethylphenoxy)-2,5-dimethylphenyl)-N-ethyl-N-methylformamidine, N'-(4-(4-fluoro-3-trifluoromethylphenoxy)-2,5-dimethylphenyl)-N-ethyl-N-methylformamidine, N'-(2-methyl-5-trifluoromethyl-4-(3-trimethylsilanylpropoxy)phenyl)-N-ethyl-N-methylformamidine, N'-(5-difluoromethyl-2-methyl-4-(3-trimethylsilanylpropoxy)phenyl)-N-ethyl-N-methylformamidine, N-methyl-(1,2,3,4-tetrahydronaphthalen-1-yl)-2-{1-[2-(5-methyl-3-trifluoromethylpyrazol-1-yl)acetyl]piperidin-4-yl}thiazole-4-carboxylate, N-methyl-(R)-1,2,3,4-tetrahyd ronaphthalen-1-yl 2-{1-[2-(5-methyl-3-trifluoromethylpyrazol-1-yl)acetyl]piperidin-4-yl}thiazole-4-carboxylate, 6-tert-butyl-8-fluoro-2,3-dimethylquinolin-4-yl acetate, 6-tert-butyl-8-fluoro-2,3-dimethylquinolin-4-yl methoxyacetate, *N*-methyl-2-{1-[2-(5-methyl-3-trifluoromethyl-1H-pyrazol-1-yl)acetyl]piperidin-4-yl}-*N*-[(1R)-1,2,3,4-tetrahydronaphthalen-1-yl]-4-thiazolecarboxamide;
G) growth regulators
   abscisic acid, amidochlor, ancymidole, 6-benzylaminopurine, brassinolide, butralin, chlormequat (chlormequat chloride), choline chloride, cyclanilid, daminozide, dikegulac, dimethipin, 2,6-dimethylpuridine, ethephon, flumetralin, flurprimidol, fluthiacet, forchlorfenuron, gibberellic acid, inabenfid, indole-3-acetic acid, maleic hydrazide, mefluidid, mepiquat (mepiquat chloride), metconazole, naphthaleneacetic acid, N-6-benzyladenine, paclobutrazole, prohexadione (prohexadione-calcium), prohydrojasmone, thidiazuron, triapenthenol, tributylphosphorotrithioate, 2,3,5-triiodobenzoic acid, trinexapac-ethyl and uniconazole;
H) herbicides
   - acetamide: acetochlor, alachlor, butachlor, dimethachlor, dimethenamid, flufenacet, mefenacet, metolachlor, metazachlor, napropamid, naproanilid, pethoxamid, pretilachlor, propachlor, thenylchlor;
   - amino acid analogs: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - bipyridyls: diquat, paraquat;
   - carbamates and thiocarbamates: asulam, butylate, carbetamide, desmedipham, dimepiperat, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bromoxynil, dichlobenil, ioxynil;
   - imidazolinones: imazamethabenz, imazamox, imazapic, imazapyr, imazaquin, imazethapyr;
   - phenoxyacetic acids: clomeprop, 2,4-dichlorophenoxyacetic acid (2,4-D), 2,4-DB, dichlorprop, MCPA, MCPA-thioethyl, MCPB, mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonylureas: amidosulfuron, azimsulfuron, bensulfuron, chlorimuron-ethyl, chlorsulfuron, cinosulfuron, cyclosulfamuron, ethoxysulfuron, flazasulfuron, fluceto-sulfuron, flupyrsulfuron, foramsulfuron, halosulfuron, imazosulfuron, iodosulfuron, mesosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propylimidazo[1,2-b]pyridazin-3-yl)sulfonyl)-3-(4,6-dimethoxypyrimidin-2-yl)urea;
   - triazines: ametryne, atrazine, cyanazine, dimethametryne, ethiozine, hexazinone, metamitron, metribuzine, prometryne, simazine, terbuthylazine, terbutryne, triaziflam;
   - ureas: chlortoluron, daimuron, diuron, fluometuron, isoproturon, linuron, methabenzthiazuron, tebuthiuron;
   - other acetolactate synthase inhibitors: bispyribac-sodium, cloransulam-methyl, diclosulam, florasulam, flucarbazone, flumetsulam, metosulam, orthosulfamuron, penoxsulam, propoxycarbazone, pyribambenz-propyl, pyribenzoxim, pyriftalide, pyriminobac-methyl, pyrimisulfan, pyrithiobac, pyroxasulfon, pyroxsulam;
   - others: amicarbazone, aminotriazole, anilofos, beflubutamid, benazolin, bencarbazone, benfluresate, benzofenap, bentazone, benzobicyclon, bromacil, bromobutide, butafenacil, butamifos, cafenstrole, carfentrazone, cinidon-ethlyl, chlorthal, cinmethylin, clomazone, cumyluron, cyprosulfamid, dicamba, difenzoquat, diflufenzopyr, *Drechslera monoceras,* endothal, ethofumesate, etobenzanid, fentrazamide, flumiclorac-pentyl, flumioxazin, flupoxam, fluorochloridon, flurtamon, indanofan, isoxaben, isoxaflutol, lenacil, propanil, propyzamide, quinclorac, quinmerac, mesotrione, methylarsenic acid, naptalam, oxadiargyl, oxadiazone, oxaziclomefon, pentoxazone, pinoxaden, pyraclonil, pyraflufen-ethyl, pyrasulfotol, pyrazoxyfen, pyrazolynate, quinoclamin, saflufenacil, sulcotrione, sulfentrazone, terbacil, tefuryltrione, tembotrione, thiencarbazone, topramezone, 4-hydroxy-3-[2-(2-methoxyethoxymethyl)-6-trifluoromethylpyridin-3-carbonyl]bicyclo[3.2.1]oct-3-en-2-one, ethyl (3-[2-chloro-4-fluoro-5-(3-methyl-2,6-dioxo-4-trifluoromethyl-3,6-dihydro-2H-pyrimidin-1-yl)phenoxy]pyridin-2-yloxy)acetate, methyl 6-amino-5-chloro-2-cyclopropylpyrimidine-4-carboxylate, 6-chloro-3-(2-cyclopropyl-6-methylphenoxy)-pyridazin-4-ol, 4-amino-3-chloro-6-(4-chlorophenyl)-5-fluoropyridin-2-carboxylic acid, methyl 4-amino-3-chloro-6-(4-chloro-2-fluoro-3-methoxyphenyl)pyridin-2-carboxylate and methyl 4-amino-3-chloro-6-(4-chloro-3-dimethylamino-2-fluorophenyl)pyridin-2-carboxylate;
I) insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoat, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, taufluvalinate, tefluthrin, tetramethrin, tralomethrin, transfluthrin, profluthrin, dimefluthrin,
   - insect growth inhibitors: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazin; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramate;
   - nicotine receptor agonists/antagonists: clothianidin, dinotefuran, imidacloprid, thiamethoxam, nitenpyram, acetamiprid, thiacloprid, 1-(2-chlorothiazol-5-ylmethyl)-2-nitrimino-3,5-dimethyl-[1,3,5]triazinane;
   - GABA antagonists: endosulfan, ethiprole, fipronil, vaniliprole, pyrafluprole, pyriprole, N-5-amino-1-(2,6-dichloro-4-methylphenyl)-4-sulfinamoyl-1 H-pyrazole-3-thiocarboxamide;
   - macrocyclic lactones: abamectin, emamectin, milbemectin, lepimectin, spinosad, spinetoram;
   - mitochondrial electron transport chain inhibitor (METI) I acaricides: fenazaquin, pyridaben, tebufenpyrad, tolfenpyrad, flufenerim;
   - METI II and III substances: acequinocyl, fluacyprim, hydramethylnone;
   - decouplers: chlorfenapyr;
   - inhibitors of oxidative phosphorylation: cyhexatin, diafenthiuron, fenbutatin oxide, propargite;
   - insect ecdysis inhibitors: cryomazin;
   - 'mixed function oxidase' inhibitors: piperonyl butoxide;
   - sodium channel blockers: indoxacarb, metaflumizon;
   - others: benclothiaz, bifenazate, cartap, flonicamid, pyridalyl, pymetrozin, sulfur, thiocyclam, flubendiamid, chlorantraniliprole, cyazypyr (HGW86); cyenopyrafen, flupyrazofos, cyflumetofen, amidoflumet, imicyafos, bistrifluron and pyrifluquinazone.

Preferred pesticides of the compositions of the present invention comprise at least one pesticide with at least one H-acidic group (such as carboxylic acid group, phosphonic acid group, phosphinic acid group) or the anionic salts thereof (e.g., mono, di or tri salts). These anionic salts of the pesticides with an H-acidic group are also suitable as anionic pesticides in group A⁻. Preferred pesticides with an H-acidic group are herbicides with an H-acidic group. Examples of herbicides with an H-acidic group are amino acid analogs (such as glyphosate or glufosinate) or imidazolinones (such as imazamethabenz, imazamox, imazapic, imazapyr, imazaquin, imazethapyr). Particularly preferred pesticides with an H-acidic group of the compositions of the present invention are glyphosate and glufosinate. In another preferred embodiment, pesticides with an H-acidic group are imidazolinones.

Especially preferably, the pesticide of the compositions of the present invention comprises a pesticide with an H-acidic group and a further pesticide. In another embodiment the pesticide of the compositions of the present invention comprises mixtures of at least two pesticides with an H-acidic group, and optionally further pesticides (such as at least one fungicide, herbicide, insecticide, and/or safener, with fungicides and/or herbicides being preferred).

In a further preferred embodiment, the pesticide of the compositions of the present invention comprises glyphosate (for example as the free acid, sodium salt, sesquisodium salt, potassium salt, dipotassium salt, ammonium salt, diammonium salt, dimethylammonium salt, trimesium salt or isopropylamine salt) or glufosinate (for example as the ammonium salt). With particular preference the pesticide of the compositions of the present invention comprises glyphosate (for example as the potassium salt, ammonium salt or isopropylamine salt). With particular preference the pesticide of the compositions of the present invention comprises glyphosate or glufosinate, and additionally a further herbicide. In another preferred embodiment the pesticide of the compositions of the present invention comprises glyphosate or glufosinate, and additionally a further pesticide (such as at least one fungicide, herbicide, insecticide and/or safener, with fungicides and/or herbicides being preferred).

The compositions according to the invention can furthermore also comprise adjuvants conventionally used for agrochemical formulations, the choice of the adjuvants depending on the specific use form, the type of formulation or the active substance. Examples of suitable adjuvants are solvents, solid carriers, surface-active substances (such as surfactants, solubilizers, protective colloids, wetters and tackifiers), organic and inorganic thickeners, bactericides, antifreeze agents, antifoams, optionally colorants and adhesives (for example for the treatment of seed) or conventional adjuvants for bait formulations (for example attractants, feedants, bittering substances).

In a preferred embodiment, the inventive composition further comprises an alkoxylate of 2-propylheptylamine. Suitable alkoxylates of 2-propylheptylamine, i.e. adjuvants used for agrochemical formulations, for use in a composition of the present invention and methods for the manufacture thereof are disclosed in WO 11/086115 and herewith incorporated by reference.

Suitable solvents are water or organic solvents such as mineral oil fractions of medium to high boiling point such as kerosene and diesel oil, furthermore coal tar oils and oils of vegetable or animal origin, aliphatic, cyclic and aromatic hydrocarbons, for example paraffins, tetrahydronaphthalene, alkylated naphthalenes and their derivatives, alkylated benzenes and their derivatives, alcohols such as methanol, ethanol, propanol, butanol and cyclohexanol, glycols, ketones such as cyclohexanone, gammabutyrolactone, dimethyl fatty acid amides, fatty acids and fatty acid esters, and strongly polar solvents, for example amines such as N-methylpyrrolidone. In principle, it is also possible to use solvent mixtures and mixtures of the abovementioned solvents and water.

Solid carriers are mineral earths such as silicas, silica gels, silicates, talc, kaolin, limestone, lime, chalk, bole, loess, clay, dolomite, diatomaceous earth, calcium and magnesium sulfate, magnesium oxide, ground synthetic materials, fertilizers such as ammonium sulfate, ammonium phosphate, ammonium nitrate, ureas and vegetable products such as cereal meal, tree bark meal, wood meal and nutshell meal, cellulose powders or other solid carriers.
Surface-active substances (adjuvants, wetters, tackifiers, dispersants or emulsifiers) which are suitable to be used in combination with the compositions of the present invention are the alkali metal, alkaline-earth metal, ammonium salts of aromatic sulfonic acids, for example of lignosulfonic acid (Borresperse^{®} types, Borregaard, Norway), phenolsulfonic acid, naphthalenesulfonic acid (Morwet^{®} types, Akzo Nobel, USA) and dibutylnaphthalenesulfonic acid (Nekal^{®} types, BASF, Germany), and of fatty acids, alkyl- and alkylarylsulfonates, alkyl ether, lauryl ether and fatty alcohol sulfates, and salts of sulfated hexa-, hepta- and octadecanols and of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl polyglycol ethers, tributylphenyl polyglycol ether, alkylaryl polyether alcohols, isotridecyl alcohol, fatty alcohol/ethylene oxide condensates, ethoxylated castor oil, polyoxyethylene or polyoxypropylene alkyl ethers, lauryl alcohol polyglycol ether acetate, sorbitol esters, lignin-sulfite liquors and proteins, denatured proteins, polysaccharides (for example methylcellulose), hydrophobe-modified starches, polyvinyl alcohol (Mowiol^{®} types, Clariant, Switzerland), polycarboxylates (Sokalan^{®} types, BASF, Germany), polyalkoxylates, polyvinylamine (Lupamin^{®} types, BASF, Germany), polyethyleneimine (Lupasol^{®} types, BASF, Germany), polyvinylpyrrolidone and their copolymers.

The composition according to the invention may comprise from 0.1 to 40% by weight, preferably from 1 to 30 and in particular from 2 to 20% by weight of surface-active substances (as disclosed above), the amount of the alkoxylate (A) and (AQ) not being taken into consideration.

Suitable thickeners are compounds which impart to the formulation a modified flow behavior, i.e. high viscosity at rest and low viscosity in the agitated state. Examples are polysaccharides, proteins (such as casein or gelatins), synthetic polymers, or inorganic layered minerals. Such thickeners are commercially available, for example Xanthan Gum (Kelzan^{®}, CP Kelco, USA), Rhodopol^{®} 23 (Rhodia, France) or Veegum^{®} (R.T. Vanderbilt, USA) or Attaclay^{®} (Engelhard Corp., NJ, USA). The thickener content in the formulation depends on the efficacy of the thickener. The skilled person will choose such a content that the desired viscosity of the formulation is obtained. The content will amount to from 0.01 to 10% by weight in most cases.

Bactericides may be added in order to stabilize the composition. Examples of bactericides are those based on diclorophene and benzyl alcohol hemiformal and also isothiazolinone derivatives such as alkylisothiazolinones and benzoisothiazolinones (Acticide^{®} MBS from Thor Chemie). Examples of suitable antifreeze agents are ethylene glycol, propylene glycol, urea and glycerol. Examples of antifoams are silicone emulsions (such as, for example, Silikon^{®} SRE, Wacker, Germany or Rhodorsil^{®}, Rhodia, France), long-chain alcohols, fatty acids, salts of fatty acids, organofluorine compounds and mixtures of these.

The composition according to the invention can preferably be present in the form of an agrochemical formulation. Examples of such formulations and their preparation are:
i) Water-soluble concentrates (SL, LS): 10 parts by weight of the active substances are dissolved using 90 parts by weight of water or a water-soluble solvent. Alternatively, wetters or other adjuvants are added. Upon dilution in water, the active substance dissolves. This gives a composition with an active substance content of 10% by weight.
ii) Dispersible concentrates (DC): 20 parts by weight of the active substances are dissolved in 70 parts by weight of cyclohexanone with addition of 10 parts by weight of a dispersant, for example polyvinylpyrrolidone. Upon dilution in water, a dispersion is obtained. The active substance content amounts to 20% by weight.
iii) Emulsifiable concentrates (EC): 15 parts by weight of the active substances are dissolved in 75 parts by weight of xylene with addition of calcium dodecylbenzenesulfonate and castor oil ethoxylate (in each case 5 parts by weight). Upon dilution in water, an emulsion is obtained. The composition has an active substance content of 15% by weight.
iv) Emulsions (EW, EO, ES): 25 parts by weight of the active substances are dissolved in 35 parts by weight of xylene with addition of calcium dodecylbenzenesulfonate and castor oil ethoxylate (in each case 5 parts by weight). Using an emulsifier (for example Ultra-Turrax), this mixture is placed into 30 parts by weight of water and made into a homogeneous emulsion. Upon dilution in water, an emulsion is obtained. The composition has an active substance content of 25% by weight.
v) Suspensions (SC, OD, FS): 20 parts by weight of the active substances are comminuted with addition of 10 parts by weight of dispersants and wetters and 70 parts by weight of water or an organic solvent in a stirred-ball mill to give a finely divided active substance suspension. Upon dilution in water, a stable suspension of the active substance is obtained. The active substance content in the composition amounts to 20% by weight.
vi) Water-dispersible and water-soluble granules (WG, SG): 50 parts by weight of the active substances are ground finely with addition of 50 parts by weight of dispersants and wetters and formulated as water-dispersible or water-soluble granules by means of technical apparatuses (for example extrusion, spray tower, fluidized bed). Upon dilution in water, a stable dispersion or solution of the active substance is obtained. The composition has an active substance content of 50% by weight.
vii) Water-dispersible and water-soluble powders (WP, SP, SS, WS): 75 parts by weight of the active substances are ground in a rotor-stator mill with addition of 25 parts by weight of dispersants and wetters and also silica gel. Upon dilution in water, a stable dispersion or solution of the active substance is obtained. The active substance content of the composition amounts to 75% by weight.
viii) Gels (GF): in a ball mill, 20 parts by weight of the active substances, 10 parts by weight of dispersant, 1 part by weight of gelling agent and 70 parts by weight of water or an organic solvent are ground to give a fine suspension. Upon dilution with water, a stable suspension with an active substance content of 20% by weight is obtained.
ix) Dusts (DP, DS): 5 parts by weight of the active substances are ground finely and mixed intimately with 95 parts by weight of finely divided kaolin. This gives a dust with an active substance content of 5% by weight.
x) Granules (GR, FG, GG, MG): 0.5 part by weight of the active substances is ground finely and associated with 99.5 parts by weight of carriers. Conventional methods to this end are extrusion, spray-drying or the fluidized bed. This gives granules for direct application with an active substance content of 0.5% by weight.
xi) ULV solutions (UL): 10 parts by weight of the active substances are dissolved in 90 parts by weight of an organic solvent, for example xylene. This gives a composition to be applied directly with an active substance content of 10% by weight.

In general, the compositions of the present invention comprise from 0.01 to 95% by weight, preferably from 0.1 to 90% by weight, of the pesticides.

The user will generally use the composition according to the invention for use in a premetering device, in a knapsack sprayer, in a spray tank or in a spraying aircraft. Here, the formulation is brought to the desired use concentration with water and/or buffer, optionally with addition of further auxiliaries, whereby the ready-to-use spray mixture (known as a tank mix) is obtained. Usually, 50 to 500 liters of the ready-to-use spray mixture are applied per hectare of utilizable agricultural area, preferably from 100 to 400 liters. In specific segments the amounts may also be above (e.g., fruit growing) or below (e.g., aircraft application) these amounts. The active substance concentrations in the ready-to-use preparations may be varied within substantial ranges. In general, they are between 0.0001 and 10%, preferably between 0.01 and 1%.

Oils of various types, wetters, drift reduction agents, stickers, spreaders, adjuvants, fertilizers, plant-strengthening products, trace elements, herbicides, bactericides, fungicides and/or pesticides may be added to the active substances or to the preparations comprising them, optionally also to the tank mix, immediately prior to use. These products can be admixed to the compositions according to the invention in the weight ratio 1:100 to 100:1, preferably 1:10 to 10:1. Adjuvants which are suitable within this context are in particular: organic-modified polysiloxanes, for example Break Thru S 240^{®}; alcohol alkoxylates, for example Atplus^{®} 245, Atplus^{®} MBA 1303, Plurafac^{®} LF 300 and Lutensol^{®} ON 30; EO/PO block polymers, for example Pluronic^{®} RPE 2035 and Genapol^{®} B; alcohol ethoxylates, for example Lutensol^{®} XP 80; and sodium dioctyl sulfosuccinate, for example Leophen^{®} RA.

Depending on the nature of the desired effect, the application rates of the active substance when used in plant protection are between 0.001 and 2.0 kg of active substance per ha, preferably between 0.005 and 2 kg per ha, especially preferably between 0.05 and 0.9 kg per ha, in particular between 0.1 and 0.75 kg per ha.

The present invention furthermore relates to a method for controlling phytopathogenic fungi and/or undesirable plant growth and/or undesirable insect or mite infestation and/or for regulating the growth of plants, wherein the composition according to the invention is allowed to act on the respective pests, the habit thereof or the plants to be protected from the respective pest, on the soil and/or on undesirable plants and/or the crop plants and/or the habitat thereof.

Examples of suitable crop plants are cereals, for example wheat, rye, barley, triticale, oats or rice; beet, for example sugar or fodder beet; pome fruit, stone fruit and soft fruit, for example apples, pears, plums, peaches, almonds, cherries, strawberries, raspberries, currants or gooseberries; legumes, for example beans, lentils, peas, lucerne or soybeans; oil crops, for example oilseed rape, mustard, olives, sunflowers, coconut, cacao, castor beans, oil palm, peanuts or soybeans; cucurbits, for example pumpkins/squash, cucumbers or melons; fiber crops, for example cotton, flax, hemp or jute; citrus fruit, for example oranges, lemons, grapefruit or tangerines; vegetable plants, for example spinach, lettuce, asparagus, cabbages, carrots, onions, tomatoes, potatoes, pumpkin/squash or capsicums; plants of the laurel family, for example avocados, cinnamon or camphor; energy crops and industrial feedstock crops, for example maize, soybeans, wheat, oilseed rape, sugar cane or oil palm; tobacco; nuts; coffee; tea; bananas; wine (dessert grapes and grapes for vinification); hops; grass, for example turf; sweetleaf (*Stevia rebaudania*); rubber plants and forest plants, for example flowers, shrubs, deciduous trees and coniferous trees, and propagation material, for example seeds, and harvested products of these plants.

The term crop plants also includes those plants which have been modified by breeding, mutagenesis or recombinant methods, including the biotechnological agricultural products which are on the market or in the process of being developed. Genetically modified plants are plants whose genetic material has been modified in a manner which does not occur under natural conditions by hybridizing, mutations or natural recombination (i.e. recombination of the genetic material). Here, one or more genes will, as a rule, be integrated into the genetic material of the plant in order to improve the plant's properties. Such recombinant modifications also comprise posttranslational modifications of proteins, oligo- or polypeptides, for example by means of glycosylation or binding of polymers such as, for example, prenylated, acetylated or farnesylated residues or PEG residues.
Examples which may be mentioned are plants which, as the result of plant-breeding and recombinant measures, have acquired a tolerance for certain classes of herbicides, such as hydroxyphenylpyruvate dioxygenase (HPPD) inhibitors, acetolactate synthase (ALS) inhibitors such as, for example, sulfonylureas (EP-A 257 993, US 5,013,659) or imidazolinones (for example US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073), enolpyruvylshikimate 3-phosphate synthase (EPSPS) inhibitors such as, for example, glyphosate (see, for example, WO 92/00377), glutamine synthetase (GS) inhibitors such as, for example, glufosinate (see, for example, EP-A 242 236, EP-A 242 246) or oxynil herbicides (see, for example, US 5,559,024). For example, breeding and mutagenesis have given rise to Clearfield^{®} oilseed rape (BASF SE, Germany), which features tolerance for imidazolinones, for example imazamox. With the aid of recombinant methods, crop plants such as soybeans, cotton, maize, beet and oilseed rape have been generated which are resistant to glyphosate or glufosinate, and these are available by the brand names RoundupReady^{®} (glyphosate-resistant, Monsanto, U.S.A.) and Liberty Link^{®} (glufosinate-resistant, Bayer CropScience, Germany).

Also comprised are plants which, with the aid of recombinant measures, produce one or more toxins, for example those from the bacterial strain *Bacillus.* Toxins which are produced by such genetically modified plants comprise, for example, insecticidal proteins of *Bacillus* spp., in particular from *B. thuringiensis,* such as the endotoxins Cry1Ab, Cry1Ac, Cry1F, Cry1Fa2, Cry2Ab, Cry3A, Cry3Bb1, Cry9c, Cry34Ab1 or Cry35Ab1; or vegetable insecticidal proteins (VIPs), for example VIP1, VIP2, VIP3, or VIP3A; insecticidal proteins from nematode-colonizing bacteria, for example *Photorhabdus* spp. or *Xenorhabdus* spp.; toxins from animal organisms, for example wasp, spider or scorpion toxins; fungal toxins, for example from Streptomycetes; plant lectins, for example from pea or barley; agglutinins; proteinase inhibitors, for example trypsin inhibitors, serine protease inhibitors, patatin, cystatin or papain inhibitors; ribosome-inactivating proteins (RIPs), for example ricin, maize RIP, abrin, luffin, saporin or bryodin; steroid-metabolizing enzymes, for example 3-hydroxysteroid oxidase, ecdysteroid IDP glycosyl transferase, cholesterol oxidase, ecdysone inhibitors or HMG CoA-reductase; ion channel blockers, for example inhibitors of sodium or calcium channels; juvenile hormone esterase; receptors for the diuretic hormone (helicokinin receptors); stilbene synthase, bibenzyl synthase, chitinases and glucanases. These toxins can also be produced, in the plants, in the form of pretoxins, hybrid proteins, truncated or otherwise modified proteins. Hybrid proteins are distinguished by a novel combination of different protein domains (see, for example, WO 2002/015701). Further examples of such toxins or genetically modified plants which produce these toxins are disclosed in EP-A 374 753, WO 93/07278, WO 95/34656, EP-A 427 529, EP-A 451 878, WO 03/18810 and WO 03/52073. The methods for generating these genetically modified plants are known to the skilled person and explained, for example, in the abovementioned publications. A large number of the abovementioned toxins impart to the plants which produce them a tolerance for pests from all taxonomic classes of the arthropods, in particular beetles (Coeleropta), dipterans (Diptera) and lepidopterans (Lepidoptera) and nematodes (Nematoda). Genetically modified plants having one or more genes which code for insecticidal toxins are described for example in the abovementioned publications and are in some cases commercially available such as, for example, YieldGard^{®} (maize varieties which produce the toxin Cry1Ab), YieldGard^{®} Plus (maize varieties which produce the toxins Cry1Ab and Cry3Bb1), Starlink^{®} (maize varieties which produce the toxin Cry9c), Herculex^{®} RW (maize varieties which produce the toxins Cry34Ab1, Cry35Ab1 and the enzyme phosphinothricin N-acetyltransferase [PAT]); NuCOTN^{®} 33B (cotton varieties which produce the toxin Cry1Ac), Bollgard^{®} I (cotton varieties which produce the toxin Cry1Ac), Bollgard^{®} II (cotton varieties which produce the toxins Cry1Ac and Cry2Ab2); VIPCOT^{®} (cotton varieties which produce a VIP toxin); NewLeaf^{®} (potato varieties which produce the toxin Cry3A); Bt-Xtra^{®}, NatureGard^{®}, KnockOut^{®}, BiteGard^{®}, Protecta^{®}, Bt11 (for example Agrisure^{®} CB) and Bt176 from Syngenta Seeds SAS, France, (maize varieties which produce the toxin Cry1Ab and the PAT enzyme), MIR604 from Syngenta Seeds SAS, France (maize varieties which produce a modified version of the toxin Cry3A, see in this context WO 03/018810), MON 863 from Monsanto Europe S.A., Belgium (maize varieties which produce the toxin Cry3Bb1), IPC 531 from Monsanto Europe S.A., Belgium (cotton varieties which produce a modified version of the toxin Cry1Ac) and 1507 from Pioneer Overseas Corporation, Belgium (maize varieties which produce the toxin Cry1 F and the PAT enzyme).

Also comprised are plants which, with the aid of recombinant measures, produce one or more proteins which bring about an increased resistance to, or ability to withstand, bacterial, viral or fungal pathogens such as, for example, so-called pathogenesis-related proteins (PR proteins, see EP-A 0 392 225), resistance proteins (for example potato varieties which produce two resistance genes against *Phytophthora infestans* from the Mexican wild potato *Solanum bulbocastanum*) or T4 lysozyme (for example potato varieties which, as the result of the production of this protein, are resistant to bacteria such as *Erwinia amylvora*).

Also comprised are plants whose productivity has been improved with the aid of recombinant methods, for example by increasing the yield potential (for example biomass, grain yield, starch content, oil content or protein content), the tolerance for drought, salt or other limiting environmental factors, or the resistance to pests and fungal, bacterial and viral pathogens.

Also comprised are plants whose constituents, in particular for improving human or animal nutrition, have been modified with the aid of recombinant methods, for example by oil plants producing health-promoting long-chain omega-3-fatty acids or monounsaturated omega-9-fatty acids (for example Nexera^{®} oilseed rape, DOW Agro Sciences, Canada).
The present invention also relates to seed (such as seeds or other plant propagation materials) comprising the composition according to the present invention. Plant propagation materials can be treated preventively with the composition according to the present invention at the point of or even before sowing or at the point of or even before transplanting. For the treatment of seed, one will generally use water-soluble concentrates (LS), suspensions (FS), dusts (DS), water-dispersible and water-soluble powders (WS, SS), emulsions (ES), emulsifiable concentrates (EC) and gels (GF). These compositions can be applied to the propagation materials, in particular seed, in undiluted form or, preferably, in diluted form. Here, the composition in question can be diluted 2- to 10-fold, so that from 0.01 to 60% by weight, preferably from 0.1 to 40% by weight, of active substance is present in the compositions used for the seed dressing. The application may be effected before or during sowing. The treatment of plant propagation material, in particular the treatment of seed, is known to the skilled person and carried out by dusting, coating, pelleting, dipping or soaking the plant propagation material, the treatment preferably being carried out by pelleting, coating and dusting or by in-furrow treatment so that, for example, untimely early germination of the seed is prevented. It is preferred to use suspensions for the treatment of seed. Usually, such compositions comprise from 1 to 800 g/l of active substance, from 1 to 200 g/l of surfactants, from 0 to 200 g/l of antifreeze agents, from 0 to 400 g/l of binders, from 0 to 200 g/l of colorants and solvent, preferably water.

The present invention furthermore relates to an alkoxylate, wherein the alkoxylate is an amine alkoxylate (A) 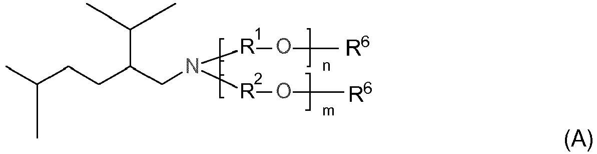 or a quaternized derivative (AQ) 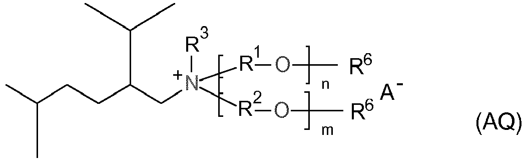 of the amine alkoxylate (A), where
- R¹, R², and R⁵: independently of one another are ethylene, propylene, butylene or a mixture of these,
- R³: is an H, -OH, -OR⁴, -[R⁵-O]ₚ-R⁶, C₁-C₆-alkyl or an oxygen anion,
- R⁴: is a C₁-C₆-alkyl, C₂-C₆-alkenyl or C₂-C₆-alkynyl,
- R⁶: is an H, C₁-C₆-alkyl, C₂-C₆-alkenyl, C₂-C₆-alkynyl, -SO₃R^{a}, -P(O)OR^{b}OR^{c}, -CH₂CO₂R^{d}, or -C(O)R^{e},
- R^{a} and R^{d}: independently of one another are an H, inorganic or organic cations,
- R^{b} and R^{c}: independently of one another are an H, inorganic or organic cations, C₁-C₆-alkyl, C₂-C₆-alkenyl or C₂-C₆-alkynyl,
- R^{e}: is C₁-C₂₂-alkyl, C₂-C₂₂-alkenyl, C₂-C₂₂-alkynyl, C₆-C₂₂-aryl or C₇-C₂₂-alkylaryl, n, m and p independently of one another have a value of from 1 to 30,
- A⁻: is an agriculturally acceptable anion, or, if R³ is an oxygen anion, A⁻ is absent.
Preferred parameters are as described above.

In one embodiment the alkoxylate is a quaternized derivative (AQ) of the amine alkoxylate (A). In a preferred embodiment, the alkoxylate is an amine alkoxylate (A).

In a further preferred embodiment, the alkoxylate is a quaternized derivative (AQ) of the amine alkoxylate (A). Here, A- is preferably a halide (such as chloride or bromide), phosphate, sulfate or an anionic pesticide. A- is especially preferably an anionic pesticide, such as glyphosate anion or glufosinate anion.

The present invention furthermore relates to methods for the production of the amine alkoxylate (A) or a quaternized derivative (AQ) of the amine alkoxylate (A), comprising the alkoxylation of 2-Isopropyl-5-methylhexane-1-amine with ethylene oxide, propylene oxide, butylene oxide or mixtures thereof. The preparation of 2-Isopropyl-5-methylhexane-1-amine is generally known, for example by reacting ammonia with 2-Isopropyl-5-methylhexanol as described for example in EP-A 696 572 or WO 2011/067199. Isopropyl-5-methylhexanol can be prepared as described in WO 2011/054483 or in the Example section below.

The alkoxylation can be catalyzed by strong bases, such as alkali metal hydroxides and alkaline earth metal hydroxides, Brönsted acids or Lewis acids, such as AlCl₃, BF₃. Catalysts such as hydrotalcite or DMC may be used for alcohol alkoxylates with a narrow distribution. The alkoxylation is preferably carried out at temperatures in the range of approximately 80 to 250°C, preferably approximately 100 to 220°C. The pressure is preferably between ambient pressure and 600 bar. If desired, the alkylene oxide may comprise an admixture of inert gas, for example of approximately 5 to 60%.

The quaternized derivative (AQ) of the amine alkoxylate (A) can be prepared in a further reaction step by quaternizing the amine alkoxylate (A). To introduce the radical R³ into the amine alkoxylate (A), the latter may be reacted for example with an alkylation reagent such as methyl chloride, dimethyl sulfate or butyl chloride. To introduce the one oxygen anion into the amine alkoxylate (A), the latter may be oxidized, for example by reacting the amino group with hydrogen peroxide, peracids (such as meta-chloroperbenzoic acid or peracetic acid) or peroxomonosulfuric acid.

The quaternized derivatives (AQ) where R³ = H can be prepared by simple protonation of starting compounds of the structure (A). The quaternized derivatives (AQ) where R³ = OH can be prepared by simple protonation of starting compounds (AQ) where R³ = oxygen anion. Acids which are suitable for the protonation are organic acids (for example C₁- to C₂₀-carboxylic acids, in particular benzoic acid) or inorganic acids (for example hydrochloric acid, phosphoric acid or sulfuric acid). Others which are likewise suitable are H-acidic pesticides such as, for example, glyphosate-acid or glyphosate-monosalts. The protonation can be carried out in a separate synthesis, so that the quaternized derivative (AQ) can be isolated. It is also possible to carry out the protonation by mixing the starting compounds with one or more acids in the composition or in the spray mixture.

The present invention also relates to the use of the amine alkoxylate (A) of the present invention or of a quaternized derivative (AQ) of the amine alkoxylate (A) of the present invention as disclosed above as adjuvants in pesticide-comprising spray mixtures. The adjuvant is preferably an activity-enhancing adjuvant. They enhance or accelerate the activity of pesticides in comparison with the activity of the pesticide in the absence of the adjuvant.

The present invention also relates to a method of improving the activity of one or more pesticides comprising the step of mixing an effective amount of amine alkoxylate (A) of the present invention or of a quaternized derivative (AQ) of the amine alkoxylate (A) of the present invention with one or more pesticides described in the present disclosure.

The advantage of the invention is the ability of the amine alkoxylate (A) of the present invention or of a quaternized derivative (AQ) of the amine alkoxylate (A) of the present invention to enhance the activity of pesticides.

The examples which follow illustrate the invention without imposing any limitation.

### Examples

### Example 1 - Synthesis of 2-Isopropyl-5-methylhexanol and 2-Isopropyl-5-methylhexane-1-amine

### a) Synthesis of 2-Isopropyl-5-methylhexanol

Under inert conditions (glove box), 102 g (1.16 mol) of isoamyl alcohol, 5.0 g (89 mmol) of KOH, 130 mg (0.46 mmol) of [Ru(COD)(Cl)₂]₂ and 250 mg (1.35 mmol) of PPh₃ were weighed into a 250 ml three-neck flask. This gave a mixture which was covered with argon. The 250 ml three-neck flask was then equipped with a reflux condenser, the mixture was heated to 100°C and stirred at 100°C for two hours. Then, 120 mg (0.48 mmol) of the ligand (VI.1.a), dissolved in 2 ml of isoamyl alcohol, were added. The reaction mixture turned brown. The brown reaction mixture was then boiled at reflux over a period of 16 hours at an oil bath temperature of 170°C using a water separator. The mixture was then also left to cool to room temperature. The gas chromatogram of the reaction mixture exhibited a conversion of isoamyl alcohol of 80.8% and a selectivity with respect to the 2-Isopropyl-5-methylhexanol of 87.2%.

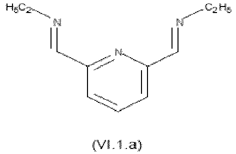

### b) Synthesis of 2-Isopropyl-5-methylhexane-1-amine

In a 9 I autoclave 315 g of 2-Isopropyl-5-methylhexanol, 1300 ml THF and 1500 g ammonia were mixed in presence of 200 ml of a solid catalyst as described in WO 2011/067199. The catalyst containing nickel, cobalt, copper, tin and aluminum was in the form of 3x3 mm tables. The autoclave was purged with hydrogen and the reaction was started by heating the autoclave. The reaction mixture was stirred for 35 hours at 195°C, the total pressure was maintained at 280 bar by purging hydrogen during the entire reductive amination step. Samples for gas chromatography were taken. After cooling down the autoclave crude product was collected, filtered, vented of excess ammonia and stripped in a rotary evaporator. The crude product was purified by distillation, a total of 265 grams of 2-Isopropyl-5-methylhexane-1-amine (colorless, 99.35% area% GC) was recovered.

**Table1: GC Samples**

| | **2-Isopropyl-5-methylhexane-1-amine [GC area%]** | **2-Isopropyl-5-methylhexanol [GC area%]** | **Others [GC area%]** |
|---|---|---|---|
| Crude product after 15 h | 73,7 | 25,6 | 0,7 |
| Crude product after 35 h | 97,2 | 1,7 | 1,1 |
| Pure product after distillation | 99,35 | 0,5 | 0,15 |

| | | | |
|---|---|---|---|
| GC-method: column 30m "RTX5 amin", program: 100-15-280/30. | | | |

### Example 2 - Alkoxylation of 2-Isopropyl-5-methylhexane-1-amine

### Preparation of 2-Isopropyl-5-methylhexane-1-amine -10 EO

110 g of 2-Isopropyl-5-methylhexane-1-amine were placed at 60°C with 8,6 g of water in an autoclave. Subsequently, the autoclave was flushed with nitrogen and the temperature was raised to 100°C. Within 1,5 hours, 62 g of ethylene oxide (EO) were metered in. The obtained reaction mixture was further stirred for 2 hours at 100°C and subsequently cooled down to room temperature.

The crude product was admixed with 1,46 g of 50% strength KOH in an autoclave and dewatering was carried out at 95°C to <20 mbar within 2 hours. The autoclave was then flushed with nitrogen, the temperature was raised to 120°C and within 3 hours, 215 g of ethylene oxide were metered in. The obtained reaction mixture was further stirred for 12 hours at 120°C and after cooling down to 80°C, remaining traces of volatile components were removed under reduced pressure. This gave 376 g of a clear yellowish liquid.
Amine value = 95 mg KOH/g
Hydroxyl value = 115 mg KOH/g

### Example 3 - Glyphosate SL formulation on wheat, soybean or maize

For the greenhouse tests, winter wheat (cultivar Cubus), soybean (cultivar Sultana), and maize (cultivar Amadeo) was sown or potted in loamy sandy soil to a depth of 1-2 cm. When the plants had reached a growth height of 10 to 25 cm (i.e., around 10 to 21 days after sowing), the spray mixtures were applied to the plants in a spraying cabin.

A concentrated formulation comprising glyphosate isopropylammonium in solution in water and amine alkoxylate from Example 2 was diluted with deionized water and applied at a water application rate of 375 I/ha (140 g of glyphosate-IPA salt/ha and 300 g of amine alkoxylate/ha). The temperatures in the experimental period, which lasted for 3 weeks, were between 18-35°C. During this time, the experimental plants received optimum watering, with nutrients being supplied via the water used for watering.

The herbicidal activity was evaluated by awarding scores to the treated plants in comparison to the untreated control plants (Table 2). The evaluation scale ranges from 0% to 100% activity. 100% activity means the complete death at least of those parts of the plant that are above ground. Conversely, 0% activity means that there were no differences between treated and untreated plants. The results in Table 2 demonstrate the increased activity of the active substance as a result of addition of the amine alkoxylate.

**Table 2: Activity [%] after 21 days**

| Amine alkoxylate | Activity [%] Winter wheat | Activity [%] Soybean | Activity [%] Maize |
|---|---|---|---|
| - ^{a)} | 18 | 20 | 32 |
| 2-Isopropyl-5-methylhexane-1-amine -10 EO | 62 | 48 | 80 |
| 2-propylheptylamine -10 EO | 55 | 42 | 80 |

| | | | |
|---|---|---|---|
| a) Comparative experiment, not inventive, without adjuvant. | | | |

### Example 4 - Glyphosate SL formulation on Sorghum and Setaria

The experiments were carried out as in Example 3, on *Sorghum halepense* (SORHA) and *Setaria verticillata* (SETVE). The application rate was 140 g of glyphosate-IPA salt/ha and 300 g of amine alkoxylate/ha. The results in Table 3 demonstrate the increased activity of the active substance as a result of addition of the amine alkoxylate.

**Table 3: Activity [%] after 21 days**

| Amine alkoxylate | Activity [%] SORHA | Activity [%] SETARIA |
|---|---|---|
| - ^{a)} | 33 | 58 |
| 2-Isopropyl-5-methylhexane-1-amine-10 EO | 93 | 78 |
| 2-propylheptylamine -10 EO | 97 | 88 |

| | | |
|---|---|---|
| a) Comparative experiment, not inventive, without adjuvant. | | |

### Example 5 - Storage stability

An aqueous formulation comprising 41.5% by weight of glyphosate-isopropylammonium salt and 15.5% by weight of 2-Isopropyl-5-methylhexane-1-amine-10 EO was storage stable for at least two weeks at temperatures of from - 5 to + 55°C.

## Claims

1. A composition comprising a pesticide and an alkoxylate, wherein the alkoxylate is an amine alkoxylate (A) 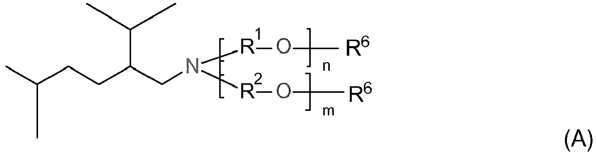 or a quaternized derivative (AQ) 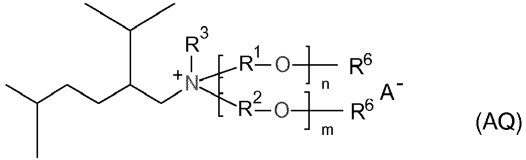 of the amine alkoxylate (A), where
R¹, R², and R⁵ independently of one another are ethylene, propylene, butylene or a mixture of these,
R³ is an H, -OH, -OR⁴, -[R⁵-O]ₚ-R⁶, C₁-C₆-alkyl or an oxygen anion,
R⁴ is a C₁-C₆-alkyl, C₂-C₆-alkenyl or C₂-C₆-alkynyl,
R⁶ is an H, C₁-C₆-alkyl, C₂-C₆-alkenyl, C₂-C₆-alkynyl, -SO₃R^{a}, -P(O)OR^{b}OR^{c}, -CH₂CO₂R^{d}, or -C(O)Re,
R^{a} and R^{d} independently of one another are an H, inorganic or organic cations,
R^{b} and R^{c} independently of one another are an H, inorganic or organic cations, C₁-C₆-alkyl, C₂-C₆-alkenyl or C₂-C₆-alkynyl,
R^{e} is C₁-C₂₂-alkyl, C₂-C₂₂-alkenyl, C₂-C₂₂-alkynyl, C₆-C₂₂-aryl or C₇-C₂₂-alkylaryl,
n, m and p independently of one another have a value of from 1 to 30,
A⁻ is an agriculturally acceptable anion, or, if R³ is an oxygen anion, A⁻ is absent.

2. The composition according to claim 1, wherein R¹, R² and R⁵ independently of one another are ethylene, ethylene and propylene, ethylene and butylene, or ethylene, propylene and butylene.

3. The composition according to claim 1 or 2, wherein A- is a halide, phosphate, sulfate or anionic pesticide.

4. The composition according to any of claims 1 to 3, wherein R³ is an H.

5. The composition according to any of claims 1 to 4, wherein, in the amine alkoxylate (A), the total of n and m is from 2 to 40, and in its quaternized derivative (AQ) the total of n, m and p is from 3 to 80.

6. The composition according to any of claims 1 to 5, wherein the alkoxylate is the amine alkoxylate (A).

7. The composition according to any of claims 1 to 6, wherein the pesticide comprises a pesticide with at least one H-acidic group.

8. The composition according to any of claims 1 to 7, wherein the pesticide comprises glyphosate or glufosinate, and additionally a further pesticide.

9. The composition according to any of claims 1 to 8, wherein the composition further comprises an alkoxylate of 2-propylheptylamine.

10. An amine alkoxylate (A) or a quaternized derivative (AQ) of the amine alkoxylate (A) according to any of claims 1 to 5.

11. A method for the production of the amine alkoxylate (A) or a quaternized derivative (AQ) of the amine alkoxylate (A) according to any of claims 1 to 5, comprising the alkoxylation of 2-Isopropyl-5-methylhexane-1-amine with ethylene oxide, propylene oxide, butylene oxide or mixtures thereof.

12. A method for controlling phytopathogenic fungi and/or undesirable plant growth and/or undesirable insect or mite infestation and/or for regulating the growth of plants, wherein the composition according to any of claims 1 to 9 is allowed to act on the respective pests, the habit thereof or the plants to be protected from the respective pest, on the soil and/or on undesirable plants and/or the crop plants and/or the habitat thereof.

13. Seed, comprising the composition according to any of claims 1 to 9.

14. Use of the amine alkoxylate (A) or a quaternized derivative (AQ) of the amine alkoxylate (A) according to any of claims 1 to 5 as adjuvants in pesticide-comprising spray mixtures.

15. The use according to claim 14, wherein the adjuvant is an activity-enhancing adjuvant.

## Patentansprüche

1. Zusammensetzung, umfassend ein Pestizid und ein Alkoxylat, wobei es sich bei dem Alkoxylat um ein Aminalkoxylat (A) 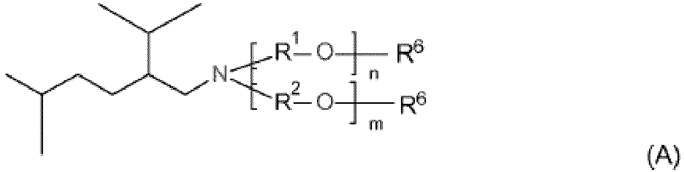 oder ein quaternisiertes Derivat (AQ) 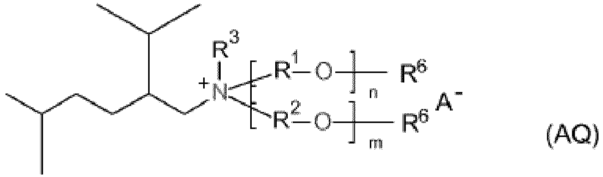 des Aminalkoxylats (A) handelt, wobei
R¹, R² und R⁵ unabhängig voneinander Ethylen, Propylen, Butylen oder eine Mischung von diesen bedeuten,
R³ H, -OH, -OR⁴, - [R⁵-O]ₚ-R⁶, C₁-C₆-Alkyl oder ein Sauerstoffanion bedeutet,
R⁴ ein C₁-C₆-Alkyl, C₂-C₆-Alkenyl oder C₂-C₆-Alkinyl bedeutet,
R⁶ ein (en) H, C₁-C₆-Alkyl, C₂-C₆-Alkenyl, C₂-C₆-Alkinyl, -SO₃R^{a}, -P(O)OR^{b}OR^{c}, -CH₂CO₂R^{d} oder -C(O)R^{e} bedeutet,
R^{a} und R^{d} unabhängig voneinander (einen) H, anorganische oder organische Kationen bedeuten,
R^{b} und R^{c} unabhängig voneinander (einen) H, anorganische oder organische Kationen, C₁-C₆-Alkyl, C₂-C₆-Alkenyl oder C₂-C₆-Alkinyl bedeuten,
R^{e} C₁-C₂₂-Alkyl, C₂-C₂₂-Alkenyl, C₂-C₂₂-Alkinyl, C₆-C₂₂-Aryl oder C₇-C₂₂-Alkylaryl bedeutet,
n, m und p unabhängig voneinander einen Wert von 1 bis 30 aufweisen,
A⁻ ein landwirtschaftlich annehmbares Anion bedeutet oder, wenn R³ ein Sauerstoffanion bedeutet, A⁻ fehlt.

2. Zusammensetzung nach Anspruch 1, wobei R¹, R² und R⁵ unabhängig voneinander Ethylen, Ethylen und Propylen, Ethylen und Butylen oder Ethylen, Propylen und Butylen bedeuten.

3. Zusammensetzung nach Anspruch 1 oder 2, wobei A⁻ ein Halogenid, Phosphat, Sulfat oder anionisches Pestizid ist.

4. Zusammensetzung nach einem der Ansprüche 1 bis 3, wobei R³ einen H bedeutet.

5. Zusammensetzung nach einem der Ansprüche 1 bis 4, wobei die Summe von n und m in dem Aminalkoxylat (A) 2 bis 40 beträgt und die Summe von n, m und p in seinem quaternisierten Derivat (AQ) 3 bis 80 beträgt.

6. Zusammensetzung nach einem der Ansprüche 1 bis 5, wobei es sich bei dem Alkoxylat um das Aminalkoxylat (A) handelt.

7. Zusammensetzung nach einem der Ansprüche 1 bis 6, wobei das Pestizid ein Pestizid mit mindestens einer sauren H-Gruppe umfasst.

8. Zusammensetzung nach einem der Ansprüche 1 bis 7, wobei das Pestizid Glyphosat oder Glufosinat und zusätzlich ein weiteres Pestizid umfasst.

9. Zusammensetzung nach einem der Ansprüche 1 bis 8, wobei die Zusammensetzung weiterhin ein Alkoxylat von 2-Propylheptylamin umfasst.

10. Aminalkoxylat (A) oder quaternisiertes Derivat (AQ) des Aminalkoxylats (A) nach einem der Ansprüche 1 bis 5.

11. Verfahren zur Herstellung des Aminalkoxylats (A) oder eines quaternisierten Derivats (AQ) des Aminalkoxylats (A) nach einem der Ansprüche 1 bis 5, bei dem man 2-Isopropyl-5-methylhexan-1-amin mit Ethylenoxid, Propylenoxid, Butylenoxid oder Mischungen davon alkyliert.

12. Verfahren zum Bekämpfen von phytopathogenen Pilzen und/oder unerwünschtem Pflanzenwuchs und/oder unerwünschtem Insekten- oder Milbenbefall und/oder zum Regulieren des Wachstums von Pflanzen, wobei man die Zusammensetzung nach einem der Ansprüche 1 bis 9 auf die jeweiligen Schädlinge, ihren Lebensraum oder die Pflanzen, die gegen den jeweiligen Schädling zu schützen sind, auf den Boden und/oder auf unerwünschte Pflanzen und/oder auf die Kulturpflanzen und/oder ihren Lebensraum einwirken lässt.

13. Saatgut, umfassend die Zusammensetzung nach einem der Ansprüche 1 bis 9.

14. Verwendung des Aminalkoxylats (A) oder eines quaternisierten Derivats (AQ) des Aminalkoxylats (A) nach einem der Ansprüche 1 bis 5 als Hilfsstoff in pestizidhaltigen Spritzbrühen.

15. Verwendung nach Anspruch 14, wobei es sich bei dem Zusatzstoff um einen wirkungsfördernden Zusatzstoff handelt.

## Revendications

1. Composition comprenant un pesticide et un alcoxylate, dans laquelle l'alcoxylate est un alcoxylate d'amine (A) 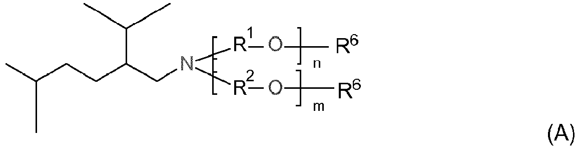 ou un dérivé quaternisé (AQ) 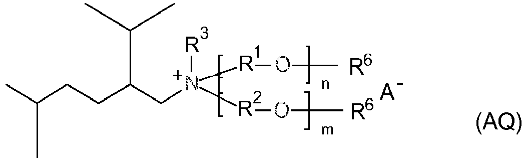 de l'alcoxylate d'amine (A), où
R¹, R² et R⁵ sont indépendamment les uns des autres éthylène, propylène, butylène ou un mélange de ceux-ci,
R³ est H, -OH, -OR⁴, - [R⁵-O]ₚ-R⁶, C₁-C₆-alkyle ou un anion d'oxygène,
R⁴ est C₁-C₆-alkyle, C₂-C₆-alcényle ou C₂-C₆-alcynyle,
R⁶ est H, C₁-C₆-alkyle, C₂-C₆-alcényle, C₂-C₆-alcynyle, -SO₃R^{a}, -P(O)OR^{b}OR^{c}, -CH₂CO₂R^{d} ou -C(O)R^{e},
R^{a} et R^{d} sont indépendamment l'un de l'autre H, des cations inorganiques ou organiques,
R^{b} et R^{c} sont indépendamment l'un de l'autre H, des cations inorganiques ou organiques, C₁-C₆-alkyle, C₂-C₆-alcényle ou C₂-C₆-alcynyle,
R^{e} est C₁-C₂₂-alkyle, C₂-C₂₂-alcényle, C₂-C₂₂-alcynyle, C₆-C₂₂-aryle ou C₇-C₂₂-alkylaryle,
n, m et p possèdent indépendamment les uns des autres une valeur allant de 1 à 30,
A⁻ est un anion acceptable sur le plan agricole, ou, si R³ est un anion d'oxygène, A⁻ est absent.

2. Composition selon la revendication 1, dans laquelle R¹, R² et R⁵ sont indépendamment les uns des autres éthylène, éthylène et propylène, éthylène et butylène, ou éthylène, propylène et butylène.

3. Composition selon la revendication 1 ou 2, dans laquelle A⁻ est un pesticide à base d'halogénure, de phosphate, de sulfate ou anionique.

4. Composition selon l'une quelconque des revendications 1 à 3, dans laquelle R³ est H.

5. Composition selon l'une quelconque des revendications 1 à 4, dans laquelle, dans l'alcoxylate d'amine (A), le total de n et m va de 2 à 40, et dans son dérivé quaternisé (AQ), le total de n, m et p va de 3 à 80.

6. Composition selon l'une quelconque des revendications 1 à 5, dans laquelle l'alcoxylate est l'alcoxylate d'amine (A).

7. Composition selon l'une quelconque des revendications 1 à 6, dans laquelle le pesticide comprend un pesticide ayant au moins un groupement acide H.

8. Composition selon l'une quelconque des revendications 1 à 7, dans laquelle le pesticide comprend du glyphosate ou du glufosinate, et en outre un autre pesticide.

9. Composition selon l'une quelconque des revendications 1 à 8, où la composition comprend en outre un alcoxylate de 2-propylheptylamine.

10. Alcoxylate d'amine (A) ou dérivé quaternisé (AQ) de l'alcoxylate d'amine (A) selon l'une quelconque des revendications 1 à 5.

11. Méthode de production de l'alcoxylate d'amine (A) ou d'un dérivé quaternisé (AQ) de l'alcoxylate d'amine (A) selon l'une quelconque des revendications 1 à 5, comprenant l'alcoxylation de 2-isopropyl-5-méthylhexane-1-amine par de l'oxyde d'éthylène, de l'oxyde de propylène, de l'oxyde de butylène, ou des mélanges de ceux-ci.

12. Méthode de contrôle de champignons phytopathogènes et/ou d'une croissance végétale indésirable et/ou d'une infestation indésirable par des insectes ou des acariens et/ou pour réguler la croissance de plantes, où on permet à la composition selon l'une quelconque des revendications 1 à 9 d'agir sur les nuisibles respectifs, l'habitat de ceux-ci ou les plantes devant être protégées contre le nuisible respectif, sur le sol et/ou sur des plantes indésirables et/ou des plantes de culture et/ou l'habitat de celles-ci.

13. Semence comprenant la composition selon l'une quelconque des revendications 1 à 9.

14. Utilisation de l'alcoxylate d'amine (A) ou d'un dérivé quaternisé (AQ) de l'alcoxylate d'amine (A) selon l'une quelconque des revendications 1 à 5, comme adjuvants dans des mélanges pour pulvérisation comprenant des pesticides.

15. Utilisation selon la revendication 14, dans laquelle l'adjuvant est un adjuvant améliorant l'activité.
